Europäisches Patentamt

European Patent Office

Office européen des brevets

(11) Publication number: **0 268 733**
**A2**

# EUROPEAN PATENT APPLICATION

(21) Application number: **87108607.0**

(51) Int. Cl.⁴: **A61M 1/00**

(22) Date of filing: **15.06.87**

(30) Priority: **27.11.86 IT 358786**

(43) Date of publication of application:
**01.06.88 Bulletin 88/22**

(84) Designated Contracting States:
**AT BE CH DE ES FR GB LI NL SE**

(71) Applicant: **DSS srl**
**Via Colombo Lolli 2**
**Ravenna(IT)**

(72) Inventor: **Bonfatti, Enrico**
**via Zagarelli alle Mura 39**
**Ravenna(IT)**

(74) Representative: **Sassatelli, Franco**
**INIP via Ruggi 5**
**I-40137 Bologna(IT)**

(54) **Surgery suction pipe.**

(57) The stainless steel pipe is particularly used in micro-surgery, and an initial cylindrical part (5) with larger section is foreseen with annular gasket for clutch binding on a beating part in the fitting seat of the sucking device. The duct rectilinear part (4) shows ahead an elbow-shaped part (8) with inside end screw thread (9) for fitting a severally shaped beak (11, 12, or 13) which shows counterposed pair of bores (17, 18 or 19) on a small cylinder forming the sucking mouth (14, 15 or 16). With no influence in the employ phasen the said bores (17, 18 or 19) act as reducers of the depression which is formed inside and avoid the accidental sucking of fragments. By equipping the base part with beaks having suited shaped and capacities, the pipe can comply with different requirements.

EP 0 268 733 A2

"Surgery suction pipe"

The invention refers to a pipe which mainly enables to obviate the inconveniences due to the occlusion of the sucking hole and, furthermore, it permits to suite the means to the different employ requirements. The invention device also improves the sealing condition and retaining in position the sucking device and avoids the wear of the means permitting the integral reinstatement also in the hot threatments and sterilization where the device is used with corrosive products. The device can particularly be used in microsurgery in which tissue and bone fragments are inserted.

In such interventions, a device with central sucking plant is required using one or more of its canals. Fur suction, one or more pipes are used to adjust by hand into the operation zone to draw the residues to remove, such as tissue brims, bone fragments, serum blood, etc..., after having been fitted on the relevant duct. At present pipes are used with permanent employ to restore by means of disencrusting and sterilizing operations, as well as others of single use type. The permanent use pipes are of the single body type and, consequently, a set of them is required with different shapes and capacities to suit the different operative requirements. In addition, they show a binding part to the sucking duct to insert by means of pressure, which forms a precarious seal. Furthermore, such pipes are manufactured in chrome-plated brass metal type and are liable to sudden degradation since they can get untrimmed and the chrome-plated layer can be stripped away; the degrading processing can then increase through hot sterilizing treatments. The single use pipes are manufactured in plastic materials: these ones are quickly degraded since they do not owe the required features to withstand hot temperatures, and even if they were in a position to withstand heat, they could not do so against the acids they might be put in contact with for disincrusting of other reasons. In any case, both above mentioned pipe types show a fundamental inconvenience because, during interventions, they might cause the accidental sucking of bone fragments, particles of tissues which are about to be inserted or other material present in the operation zone, on account of the occlusion of the sucking mouth which suddenly increases the duct inside depression.

This is obviated by the invention device which permits the instantaneous intervention when the suction effect is rising, thus avoiding that dangerous limits are reached. The tool consists of a base part initially equipped with a device for imposing it on the duct of the sucking device which can be equipped with a set of phial necks with different shapes and capacities, to be singularly screwed as a duct prosecution on the other end, thus allowing each time to suit with the operation requirements. The two above mentioned pipe components are preferably manufactured in stainless steel, as thus their degradation can be avoided. The base is foreseen in an initial cylindrical part of larger section on a duct rectilinear part, equipped with an anular gasket, to be introduced into a corresponding seat of the sucking duct of the central device. This imposing device carries out a binding inserting condition on the beating part, thus ensuring the seal and an eficient retention effect against unbinding. The duct rectilinear part is foreseen with an elbow-shaped piece with inside end threading for screwing a small beak on it which shows a pair of bores on a small cylinder forming the minimum section sucking mouth. Without any influence at all in conventional employ phase, these bores act as reducers of the inside depression which is formed in the duct through the obstruction effect, thus avoiding the accidental suction of bone fragments, tissue particles to be inserted or other just present in the operation zone.

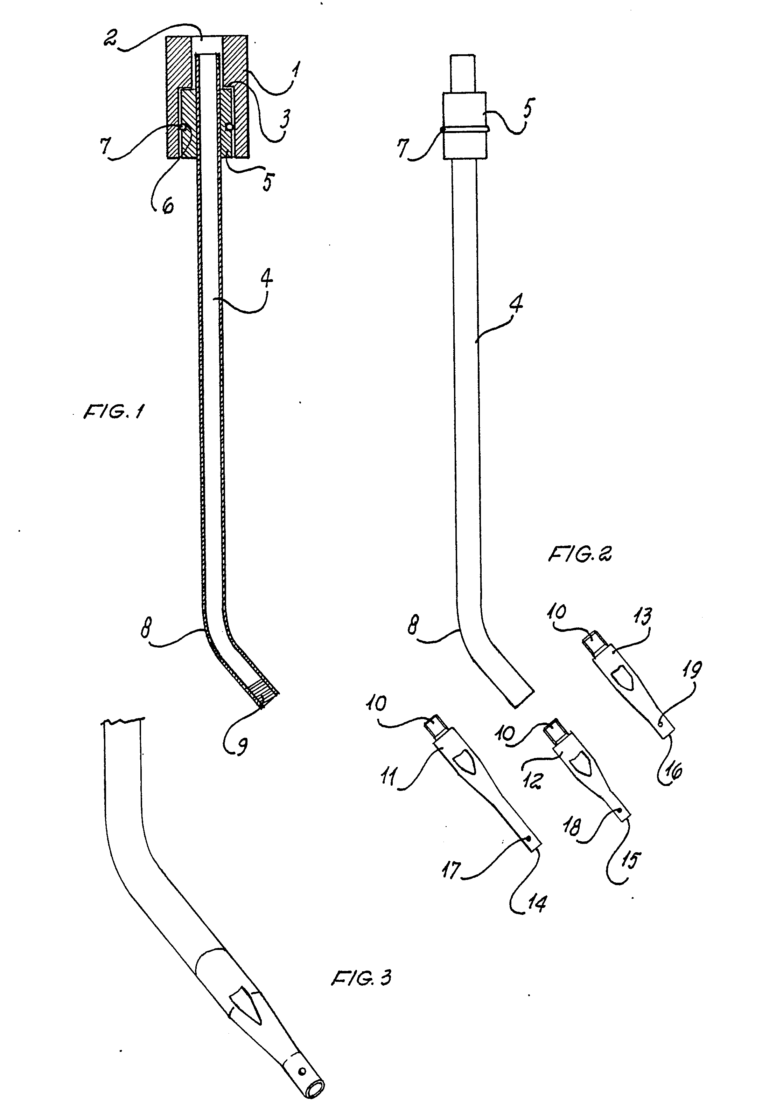

Form of execution is shown in an indicative way in the drawings of table 1. With reference to this one, fig. 1 is the longitudinal section of the pipe fitted on a sucking duct. Fig. 2 is the side view of a pipe fitted with three beaks of different shapes and contents. Fig. 3 is the end view of a pipe with a beak fitted on it.

The invention foresees the part of sucking duct 1 with duct 2 fitted with beating crown 3. The pipe consists of rectilinear duct 4 and iniitial integral cylindrical part 5 with hollow 6 for positioning OR 7. The pipe is further foreseen with an elbow 8 having an inside screw thread 9 for fitting on the axial part 10 corresponding to the thread of one of beaks 11, 12 and 13. On the end small cylinders of the said beaks with delivery openings 14, 15 and 16, the counterposed pairs of bores 17, 18 and 19 are foreseen.

The shapes of the fitting devices, the relevant gaskets, the shape of the duct portion of the pipe, the beaks and relevant assembling means of the same on the base body, as well as the materials may be foreseen in different ways.

**Claims**

1) Surgery suction pipe, characterized by the fact it consists of a base part initially equipped with a device for fitting it on the duct part. This base part is foreseen with an initial cylindrical part (5) with larger section, on the rectilinear part (4), to introduce into a corresponding suction duct of the device with central supply system. The head of the duct part (4) shows an elbow-shaped part (8) with inside end screw thread (9) for fitting several types of beaks (11, 12 or 13). As fundamental feature, these beaks show a counterposed pair of bores (17, 18 or 19) with minimum section. Without any influence in the employ phase, the above mentioned bores (17, 18 and 19) act as reducers of the inside depression, thus avoiding the accidental suction of fragments.

0 268 733

FIG.1

FIG.2

FIG.3